# EUROPÄISCHE PATENTSCHRIFT

(11) **EP 0 900 777 B1**
(45) Veröffentlichungstag und Bekanntmachung des Hinweises auf die Patenterteilung: **10.04.2002**
(21) Anmeldenummer: 98113771.4
(22) Anmeldetag: 23.07.1998
(51) Int. Cl.: C07C 67/08, C07C 69/74

(54) **Verfahren zur Herstellung von Cyclopropancarbonsäureestern von C1-C4 Alkoholen**
Process for the preparation of esters of cyclopropanecarboxylic acid with C1-C4 alcohols
Procédé de fabrication d'esters de l'acide cyclopropanecarboxylique avec des alcools en C1-C4

(30) Priorität: 01.09.1997 DE 19738072
(43) Veröffentlichungstag der Anmeldung: 10.03.1999
(73) Patentinhaber: Degussa AG, 40474 Düsseldorf (DE)
(72) Erfinder: Kaufhold, Manfred, Dr., 45770 Marl (DE); Feld, Marcel, Dr., 51147 Köln (DE)

(56) Entgegenhaltungen:
- US-A- 5 504 245

## Beschreibung

Die Erfindung betrifft ein Verfahren zur Herstellung von Cyclopropancarbonsäureestem von Alkoholen mit 1 bis 4 Kohlenstoffatomen durch Veresterung der Carbonsäuren mit diesen Alkoholen,

Cyclopropancarbonsäureester niederer Alkohole haben in letzter Zeit als Zwischenprodukte im Pharma- und Agrochemikalienbereich erhebliche Bedeutung erlangt. Dazu hat beigetragen, daß Cyclopropancarbonsäure in neuerer Zeit durch Oxidation des entsprechenden Aldehyds gut zugänglich geworden ist (siehe US-A 5504245) und großtechnisch hergestellt wird. Bis heute gibt es jedoch kein befriedigendes Verfahren zur Herstellung dieser Ester.

Die Veresterung von Carbonsäuren mit einem Alkohol unter Freisetzung von Wasser ist eine bekannte Reaktion, die zu einem Gleichgewicht führt. Die Einstellung des Gleichgewichts wird in der Regel durch saure Katalysatoren beschleunigt. Durch Entfernung des Reaktionswassers mit Hilfe eines Schleppmittels und/oder Verwendung eines Überschusses an Alkohol kann das Gleichgewicht in Richtung auf die Esterbildung verschoben werden. Dieses Prinzip wird bei dem Verfahren nach der Patentschrift SU 322 986 angewandt, bei dem man Cyclopropancarbonsäure bei ca. 80°C mit einem großen Überschuß an Methanol in Gegenwart von p-Toluolsulfonsäure als Katalysator und von Toluol oder Tetrachlorkohlenstoff als Schleppmittel für das Wasser verestert. Dabei ist jedoch die Ausbeute mit 70% unbefriedigend. Zudem wird durch das Schleppmittel ein weiterer Stoff in das Verfahren eingeführt.

Kohlrausch und Skrabal (Monatsh.Chem. **70** [1937], 377,395) stellten den Cyclopropancarbonsäuremethylester durch Veresterung der Säure mit Methanol unter Verwendung von konzentrierter Schwefelsäure als Katalysator her. Der so erhaltene Rohester mußte jedoch mehrmals destilliert werden, um ein reines Produkt zu erhalten. Jede zusätzliche Destillation erhöht den Aufwand und vermindert die Ausbeute.

Folmer und Weinreb (Tetrahedron Lett. [1993] **34** (17). 2737-40) erzielten relativ gute Ausbeuten an Cyclopropancarbonsäuremethylester durch Veresterung bei Temperaturen von -78°C bis Raumtemperatur, verwendeten aber mit dem sog. Appel-Salz (4,5-Dichlor-1.2,3-dithiazolium-chlorid) sowie mit 2,6-Dimethylpyridin teure und schwierig zu entsorgende Hilfsstoffe.

Diese und weitere in der Literatur beschriebene Verfahren arbeiten bei Temperaturen unter 100°C (z.B. Saigo, Kazihiko Saigo, Masahiro Usui, Kazumori Kikuchi, Eiichiro Shimada und Teruaki Mukayama. Bull. Chem. Soc. Jpn. [1977] **50**(7), 1863-6 bei Raumtemperatur) und tragen damit der bekannten thermischen Instabilität von Cyclopropanverbindungen Rechnung (siehe z.B. Hans Beyer, Lehrbuch der Organischen Chemie, S.Hirzel Verlag, Leipzig. 15./16. Auflage (1968). Seite 357). Die bekannten Verfahren arbeiten zumeist mit einem Überschuß an Alkohol in der Veresterungszone. Die Verfahren haben unbefriedigende Ausbeuten, unvorteilhaft viele Stufen und/oder benötigen teure und schwierig zu entsorgende Hilfsstoffe.

Aufgabe der Erfindung war es daher, ein Verfahren bereitzustellen, das gute Ausbeuten ergibt, mit wohlfeilen Hilfsstoffen arbeitet und ein Produkt ergibt, das auf einfache Weise gereinigt werden kann.

Diese Aufgabe wird gelöst durch ein Verfahren zur Herstellung von Cyclopropancarbonsäureestern von Alkoholen mit 1 bis 4 Kohlenstoffatomen durch Veresterung der entsprechenden Cyclopropancarbonsäuren mit den entsprechenden Alkoholen in Gegenwart eines sauren Katalysators, bei dem man in der Veresterungszone einen 2- bis 1.000-fachen stöchiometrischen Überschuß der Cyclopropancarbonsäure über dem Alkohol aufrechterhält, die Temperatur in der Veresterungszone auf 100 bis 200 °C hält und den Cyclopropancarbonsäureester zusammen mit dem Reaktionswasser und kleinen Mengen Alkohol aus der Reaktionszone abdestilliert.

Das erfindungsgemäße Verfahren ergibt hochreinen Ester in Ausbeuten von mehr als 90%, bezogen auf umgesetzte Cyclopropancarbonsäure. Überraschenderweise muß man nicht unter schonenden Bedingungen, d.h. bei Temperaturen <100°C arbeiten. Die gegen thermische Beanspruchung insbesondere in saurem Milieu empfindliche Cyclopropancarbonsäure wird, da sie in erheblichem Überschuß vorliegt, beträchtlich länger diesen belastenden Bedingungen ausgesetzt als der insoweit unproblematische Alkohol. Trotzdem kommt es nicht zu Nebenreaktionen, und Zersetzungsprodukte werden nicht einmal in Spuren gefunden. Wegen der hohen Temperaturen erzielt man zudem hervorragende Raum-Zeit-Ausbeuten. Das Verfahren kann absatzweise oder, mit besonderem Vorteil, kontinuierlich durchgeführt werden.

Mit dem Verfahren nach der Erfindung lassen sich die unsubstituierte Cyclopropancarbonsäure oder deren im Ring substituierte Derivate umsetzen, z.B. die durch 1 oder 2 Alkylreste mit 1 bis 4 Kohlenstoffatomen ringsubstituierten Cyclopropancarbonsäuren.

Unter den Alkoholen werden Ethanol, n-Propanol, i-Propanol, n-Butanol und insbesondere Methanol bevorzugt.

Als Veresterungskatalysatoren eignen sich die bekannten sauren Katalysatoren, wie Schwefelsäure; Sulfonsäuregruppen enthaltende Ionenaustauschharze; Sulfonsäuren, wie Methansulfonsäure, höhere Alkansulfonsäuren, Benzolsulfonsäure, p-Toluolsulfonsäure und insbesondere höhere Alkylbenzolsulfonsäuren, vorteilhaft solche mit gegebenenfalls verzweigten C₁₀-bis C₁₃-Alkylresten. Man wendet die Katalysatoren im allgemeinen in solchen Mengen an, daß ihr Anteil in der Veresterungszone 0,1 bis 10 Gewichtsprozent beträgt.

Das Verfahren nach der Erfindung wird vorteilhaft bei einer Temperatur von 120 bis 200°C, insbesondere bei 130 bis 150°C durchgeführt. Man arbeitet im allgemeinen bei Atmosphärendruck. Erhöhter Druck empfiehlt sich, wenn man bei einer Temperatur arbeiten will, bei der die Cyclopropancarbonsäure unter Atmosphärendruck gasförmig ist (die unsubstituierte Cyclopropancarbonsäure siedet bei 182°C). Verminderter Druck kann dann angewandt werden, wenn man einen relativ hochsiedenden niederen Alkohol, wie n-Propanol einsetzt.

Ein wichtiges Merkmal des erfindungsgemäßen Verfahrens besteht darin, daß in der Veresterungszone ein erheblicher stöchiometrischer Überschuß an Cyclopropancarbonsäure über den niederen,Alkohol vorliegt. Die Cyclopropancarbonsäure liegt in der 2 bis 1.000-fachen, vorteilhaft in der 10- bis 200-fachen stöchiometrischen Menge vor. Bei absatzweiser Durchführung des Verfahrens kann man z.B. niederen Alkohol nach und nach allein in die vorgelegte Cyclopropancarbonsäure einführen oder aber in Form eines Gemisches mit weiterer Cyclopropancarbonsäure, wobei deren Anteil geringer ist, als dem in der Veresterungszone entstehenden Ester entspricht. Die vorgelegte Cyclopropancarbonsäure wird in beiden Fällen mehr oder weniger schnell aufgebraucht. Mit dem Reaktionswasser abdestillierender niederer Alkohol kann in die Veresterung zurückgeführt werden. Bei kontinuierlicher Arbeitsweise legt man wiederum Cyclopropancarbonsäure vor und führt kontinuierlich eine Mischung aus Cyclopropancarbonsäure und niederem Alkohol zu, wobei das Mischungsverhältnis so gewählt werden kann, daß die zugeführte Menge Cyclopropancarbonsäure dem in der Reaktionszone entstehenden und abdestillierenden Ester entspricht, so daß in der Reaktionszone stationärer Zustand herrscht. Das ist im allgemeinen der Fall, wenn das Molverhältnis von Cyclopropancarbonsäure und niederem Alkohol. je nach den Einsatzstoffen, im Bereich von 1 : 1,20 bis 1 : 1,02, vorteilhaft von 1 : 1,10 bis 1 : 1,05 liegt. Der geringe Überschuß an niederem Alkohol destilliert mit dem gebilden Ester und dem Reaktionswasser ab.

Das Dampfgemisch wird kondensiert, und das Kondensat bildet zwei Phasen. Die obere, wäßrige Phase kann zur Rückgewinnung des darin enthaltenen niederen Alkohols andestilliert und der so rückgewonnene niedere Alkohol in die Veresterung zurückgeführt werden. Die weitere Destillation der wäßrigen Phase ergibt geringe Mengen an Ester, der nach Abtrennung von dem azeotrop mitdestillierenden Wasser ebenfalls in die Veresterung zurückgeführt oder zusammen mit der unteren, organischen Phase aufgearbeitet werden kann. Der Rückstand ist Abwasser mit wenig Verunreinigungen, das leicht entsorgt werden kann.

Die untere, organische Phase wird in analoger Weise andestilliert und dadurch von geringen Mengen Wasser und niederem Alkohol befreit. Der Rückstand ist sehr reiner Cyclopropancarbonsäureester, der für die meisten Verwendungen nicht mehr destilliert zu werden braucht.

Die folgenden Beispiele sollen das erfindungsgemäße Verfahren weiter erläutern, nicht aber seinen Anwendungsbereich begrenzen, wie er in den Patentansprüchen definiert ist. Alle Gehalte und Prozentgehalte wurden gaschromatographisch bestimmt.

### Beispiel 1 - Cyclopropancarbonsäuremethylester

Man benutzt eine Glasapparatur, die aus einem Fünfhalskolben mit Rührer, Thermometer, Tropftrichter, Destillationskolonne mit aufgesetzter Destillationsbrücke und Einleitungsrohr bis zum tiefsten Punkt im Kolben besteht.

Man legt in dem Kolben 258,3 g (3 mol) Cyclopropancarbonsäure (CPC) sowie 10 g C₁₀-C₁₃-Alkylbenzolsulfonsäure (Marlon^{(R)}AS3-Säure) vor und erhitzt das Gemisch auf 140°C. Dann werden über das Einleitungsrohr stündlich 60 ml einer Mischung aus 430,5 g (5 mol) Cyclopropancarbonsäure und 173 g (5,4 mol) Methanol zugegeben. Diese Mengen an Einsatzstoffen entsprechen nach 13 Stunden etwa den Mengen an abdestillierende Stoffen, so daß die Standhöhe dann nahezu konstant bleibt.

Es werden 403 g Destillat erhalten, das in zwei Phasen zerfällt, die getrennt werden.

Die obere, wäßrige Phase, 88 g, hat folgende Zusammensetzung:

| | |
|---|---|
| Wasser | 70,0 Gew.-% |
| Methanol | 24,4 Gew.% |
| Cyclopropancarbonsäuremethylester (CPME) | 5,4 Gew.% |

Die untere, organische Phase, 315 g, hat folgende Zusammensetzung:

| | |
|---|---|
| Wasser | 6,8 Gew -% |
| Methanol | 7,1 Gew.-% |
| CPME | 86,0 Gew.-% |

Im Reaktionskolben verbleiben 455 g mit folgender Zusammensetzung:

| | |
|---|---|
| Wasser | 0,6 Gew.-% |
| CPME | 29,1 Gew.-% |
| CPC und Alkylbenzolsulfonsäure | 70,1 Cew.-% |

Die Ausbeute an CPME, bezogen auf umgesetzte CPC, beträgt 91 % d.Th. Zur Reinigung des rohen CPME (315 g) wird die organische Phase nur andestilliert und dadurch Wasser sowie Methanol abgetrennt, das in die Veresterung zurückgeführt werden kann. Als Rückstand fällt CPME in einer Reinheit von 99.8 % an.

Die wäßrige Phase wird ebenfalls nur andestilliert und dadurch CPME und Methanol gewonnen. Als Sumpf verbleibt ein leicht zu entsorgendes Abwasser mit einem Kohlenstoffgehalt von 400 mg/l

### Beispiel 2 - Cyclopropancarbonsäureethylester

Man benutzt die in Beispiel 1 beschriebene Apparatur und setzt ein:

| | |
|---|---|
| 258,3 g (3 mol) | Cyclopropancarbonsäure |
| 7 g | C₁₀₋₁₃-Alkylbenzolsulfonsäure (Marlon^{(R)} AS 3-Säure) |

Die Stoffe werden zusammengegeben und auf 140°C erwärmt. Bei dieser Temperatur wird eine Mischung aus 733 g (8,51 mol) CPC und 423,6 g (9,19 mol) Ethanol mit einer Dosierung von 100 ml/h zugepumpt. Die Standhöhe steigt zunächst an, da die Menge des Destillats geringer als die zugepumpte Menge ist. Durch Erhöhung der Temperatur auf 160°C wird ein stationärer Zustand eingestellt. Es fallen homogene Destillate mit unterschiedlichem Wassergehalt an:

| | | | |
|---|---|---|---|
| 1. | Destillat | 257 g | 20,7 Gew.-% Wasser |
| 2. | Destillat | 275 g | 12,7 Gew.-% Wasser |
| 3. | Destillat | 162 g | 0,5 Gew.-% Wasser |

Die Destillate 1 und 2 werden zusammengegeben, und Ethanol wird als Azeotrop mit Wasser abdestilliert. Der Destillationsrückstand besteht aus zwei Phasen, die sich nach Zugabe von 7 g Natriumsulfat leicht trennen lassen. Die untere, wäßrige Phase wird verworfen. Die obere Phase, 254,8 g, ist reiner Cyclopropancarbonsäureethylester. Aus dem Reaktorsumpfprodukt werden durch Ausdestillieren weitere 1,61 mol Ester gewonnen. 5,68 mol CPC verbleiben als Rückstand.

Insgesamt werden 5,22 mol Ester erhalten. Die Ausbeute beträgt 89,5% d.Th., bezogen auf umgesetzte CPC.

### Beispiel 3 - Cyclopropancarbonsäure-n-propylester

Man benutzt die in Beispiel 1 beschriebene Apparatur und setzt ein:

| | |
|---|---|
| 317 g (3,68 mol) | Cyclopropancarbonsäure |
| 10 g | C₁₀₋₁₃-Alkylbenzolsulfonsäure (Marlon^{(R)} AS 3-Säure) |

Die Stoffe werden zusammengegeben und auf 170°C erwärmt. Bei dieser Temperatur wird eine Mischung aus 528.3 q (7.13 mol) CPC und 462,8 g (7,7 mol) n-Propanol innerhalb von 10 Stunden zugepumpt Bei der gewählten hohen Temperatur von 170°C steigt die Standhöhe im Reaktor nicht an. Das angefallene Destillat hat die folgende Zusammensetzung:

| | |
|---|---|
| 89,9 Gew.-% | CPC-n-Propylester |
| 3,2 Gew.-% | CPC |
| 6,4 Gew.-% | n-Propanol |

In diesem Fall findet sich also eine relativ große Menge CPC im Destillat, was auf die hohe Sumpftemperatur zurückzuführen ist. Die Aufarbeitung erfolgt wie in Beispiel 2 beschrieben. Man erhält den CPC-n-Propylester in einer Reinheit von 99,8 Gew.-% mit einer Ausbeute von ca. 81 % d.Th., bezogen auf umgesetzte CPC.

## Patentansprüche

1. Verfahren zur Herstellung von Cyclopropancarbonsäureestern von Alkoholen mit 1 bis 4 Kohlenstoffatomen durch Veresterung der entsprechenden Cyclopropancarbonsäuren mit den entsprechenden Alkoholen in Gegenwart eines sauren Katalysators,
**dadurch gekennzeichnet,**
**dass** man in der Veresterungszone einen 2 bis 1.000-fachen stöchiometrischen Überschuß der Cyclopropancarbonsäure über dem Alkohol aufrechterhält, die Temperatur in der Veresterungszone auf 100 bis 200 °C hält und den Cyclopropancarbonsäureester zusammen mit dem Reaktionswasser und kleinen Mengen Alkohol aus der Reaktionszone abdestilliert.

2. Verfahren nach Anspruch 1,
**dadurch gekennzeichnet,**
**dass** die Temperatur in der Veresterungszone 120 bis 200 °C beträgt.

3. Verfahren nach Anspruch 1,
**dadurch gekennzeichnet,**
**dass** die Temperatur in der Veresterungszone 130 bis 150 °C beträgt.

4. Verfahren nach einem der Ansprüche 1 bis 3,
**dadurch gekennzeichnet,**
**dass** als saurer Katalysator Schwefelsäure, ein Sulfonsäuregruppen enthaltendes Ionenaustauschharz oder eine Sulfonsäure verwendet wird.

5. Verfahren nach Anspruch 4,
**dadurch gekennzeichnet,**
**dass** als Sulfonsäure eine höhere Alkylbenzolsulfonsäure mit gegebenenfalls verzweigten C₁₀- bis C₁₃-Alkylresten verwendet wird.

## Claims

1. A process for preparing a cyclopropanecarboxylate of an alcohol having 1 to 4 carbon atoms by esterification of the corresponding cyclopropanecarboxylic acid with the corresponding alcohol in the presence of an acidic catalyst, **characterized in that** in the esterification zone a 2 to 1 000-fold stoichiometric excess of the cyclopropanecarboxylic acid with respect to the alcohol is maintained, the temperature in the esterification zone is kept at from 100 to 200°C and, from the reaction zone, the cyclopropanecarboxylate is distilled off together with the water of reaction and small amounts of alcohol.

2. A process according to claim 1, **characterized in that** the temperature in the esterification zone is from 120 to 200°C.

3. A process according to claim 1, **characterized in that** the temperature in the esterification zone is from 130 to 150°C.

4. A process according to any one of claims 1 to 3, **characterized in that** the acid catalyst used is sulphuric acid, an ion exchange resin containing sulphonic acid groups or a sulphonic acid.

5. A process according to claim 4, **characterized in that** the sulphonic acid used is a higher alkylbenzenesulphonic acid having branched or unbranched C₁₀- to C₁₃-alkyl radicals.

## Revendications

1. procédé de préparation d'esters d'acide cyclopropanecarboxylique d'alcools ayant de 1 à 4 atomes de carbone, par estérification des acides cyclopropanecarboxyliques correspondants avec les alcools correspondants en présence d'un catalyseur acide,
**caractérisé en ce qu'**
on maintient dans la zone d'estérification un excès de 2 à 1000 fois par rapport à la stoechiométrie d'acide cyclopropanecarboxylique par rapport à l'alcool, on conserve la température dans la zone d'estérification de 100 à 200°C et on sépare par distillation de la zone de réaction l'ester d'acide cyclopropanecarboxylique conjointement avec l'eau de la réaction et de petites quantités d'alcool.

2. Procédé selon la revendication 1,
**caractérisé en ce que**
la température dans la zone d'estérification s'élève de 120 à 200°C.

3. Procédé selon la revendication 1,
**caractérisé en ce que**
la température dans la zone d'estérification s'élève à 130 à 150°C.

4. Procédé selon la revendication 1,
**caractérisé en ce que**
comme catalyseur acide on utilise de l'acide sulfurique, une résine échangeuse d'ions contenant des groupes acide sulfonique ou un acide sulfonique.

5. procédé selon la revendication 4,
**caractérisé en ce que**
comme acide sulfonique on utilise un acide alkylbenzènesulfonique supérieur ayant des restes alkyle en C₁₀-C₁₃ éventuellement ramifiés.
